# EUROPÄISCHE PATENTSCHRIFT

(11) **EP 0 437 183 B1**
(45) Veröffentlichungstag und Bekanntmachung des Hinweises auf die Patenterteilung: **27.07.1994**
(21) Anmeldenummer: 90811024.0
(22) Anmeldetag: 21.12.1990
(51) Int. Cl.: A61N 5/06

(54) **Lichtdiffusor für eine photodynamische Therapie von Tumoren im Oesophagus eines Patienten**
Light diffuser for a photodynamic therapy of tumours in the oesophagus of a patient
Diffuseur de lumière pour thérapie photodynamique de tumeurs dans l'oesophage d'un patient

(30) Priorität: 09.01.1990 CH 60/90
(43) Veröffentlichungstag der Anmeldung: 17.07.1991
(73) Patentinhaber: CIBA-GEIGY AG, 4002 Basel (CH)
(72) Erfinder: Wagnières, Georges, CH-1095 Lutry (CH); van den Bergh, Hubert, Dr., CH-1376 Goumoens-la-Ville (CH); Monnier, Philippe, Dr., CH-1010 Lausanne (CH)

(56) Entgegenhaltungen:
- WO-A-85/05262
- GB-A- 2 154 761
- US-A- 4 660 925
- US-A- 4 693 556

## Beschreibung

Die Erfindung betrifft einen Lichtdiffusor für eine photodynamische Therapie von Tumoren im Oesophagus eines Patienten mit einer optischen Faser zum Einspeisen von Laserlicht in ein das axial einfallende Licht radial umlenkende und in eine Masse eingebettete Partikel enthaltenden Rohr, das an dem der optischen Faser gegenüberliegenden vorderen Ende einen Spiegel aufweist und koaxial in einem hülsenförmigen Sondengehäuse angeordnet ist.

Ein derartiger Lichtdiffusor ist aus Hubert van den Bergh, Light and porphyrins in cancer therapy, Chemistry in Britain, Mai 1986, Vol. 22, Nr. 5 bekannt und verfügt an seinem vorderen Ende über einen Savary-Gillard Dilator, der ein schonendes Einführen des einen Durchmesser von etwa 20 mm aufweisenden Sondengehäuses mit einer Gesamtlänge von etwa 75 mm gestattet. Das hülsenförmige Sondengehäuse besteht aus PMMA und ist mit dem vorderen Ende eines Nylonschlauches verbunden, der mit Abstandsmarkierungen versehen ist. Durch das Lumen des Nylonschlauches erstreckt sich die optische Faser und endet im Innern des Sondengehäuses in einem geringen Abstand von dem das Licht in radialer Richtung verteilenden Rohr. Das Rohr besteht aus PTFE und ist mit Epoxydharz gefüllt, dem TiO₂-Partikel mit einer Konzentration von 0,1 bis 1 Prozent zugefügt sind. Dabei weist der erste Abschnitt, der der optischen Faser am nächsten liegt, eine mittlere Konzentration von 0,5 Prozent TiO₂ auf. Der mittlere Abschnitt des Rohres hat eine Konzentration von 0,1 Prozent und der letzte Abschnitt eine Konzentration von 1 Prozent TiO₂-Partikel. Der Durchmesser der Partikel beträgt 0,2 µm, so dass sich im Hinblick auf die Lichtwellenlänge in der gleichen Grössenordnung eine Debyestreuung ergibt, die stark wellenlängenabhängig ist.

Der Erfindung liegt die Aufgabe zugrunde, einen Lichtdiffusor der eingangs genannten Art zu schaffen, der älterungsbeständig ist und eine möglichst homogene Dosierung der für die photodynamische Therapie erforderlichen Strahlung sowie der für eine Hyperthermie notwendigen längerwelligen Strahlung gestattet.

Diese Aufgabe wird erfindungsgemäss dadurch gelöst, dass die das Licht umlenkenden Partikel Körner mit einem gegenüber der Lichtwellenlänge grossen Durchmesser und einem vom Brechungsindex der Masse verschiedenen Brechungsindex sind.

Bei einem bevorzugten Ausführungsbeispiel der Erfindung sind die Körner Quarzkörner mit einem Durchmesser von etwa 40 µm und die Masse besteht aus Silikon.

Um eine möglichst homogene Lichtverteilung im Oesophagus zu erhalten, ist das Rohr in axialer Richtung in aufeinanderfolgende Abschnitte mit unterschiedlichen Konzentrationen für die Quarzkörner ausgestaltet. Dabei hat der vom Laserlicht direkt beaufschlagte erste Abschnitt eine grössere Quarzkörnerkonzentration als die in axialer Richtung zum vorderen Ende nachfolgenden Abschnitte.

Vorzugsweise schliesst sich in axialer Richtung an den ersten Abschnitt hoher Quarzkörnerkonzentration wenigstens ein Abschnitt mit einer kleineren Quarzkörnerkonzentration und in axialer Richtung daran anschliessend ein letzter Abschnitt mit mittlerer Quarzkörnerkonzentration an.

Besonders zweckmässig ist es, wenn zwischen dem ersten Abschnitt und dem letzten Abschnitt zwei Abschnitte für sehr kleine und Kleine Quarzkörnerkonzentration als mittlerer Bereich vorgesehen sind.

Um eine gezielte Hyperthermiebehandlung durchführen zu können, ist im Mantel des Sondengehäuses ein Thermoelement vorgesehen, das an eine thermische Ueberwachungseinrichtung angeschlossen ist.

Vorzugsweise ist zusätzlich zu der für die Lichteinspeisung vorgesehenen optischen Faser eine Lichtüberwachungsfaser vorgesehen, deren Stirnfläche seitlich versetzt neben der optischen Faser angeordnet ist und mit einem Teil des von dem hinteren Ende des Rohres zurückkehrenden Lichtes beaufschlagt ist. Die Lichtüberwachungsfaser ist an eine Lichtintensitätssteuer- und Ueberwachungseinrichtung angeschlossen und ermöglicht somit eine genaue Ueberwachung und Steuerung der photodynamischen Therapie.

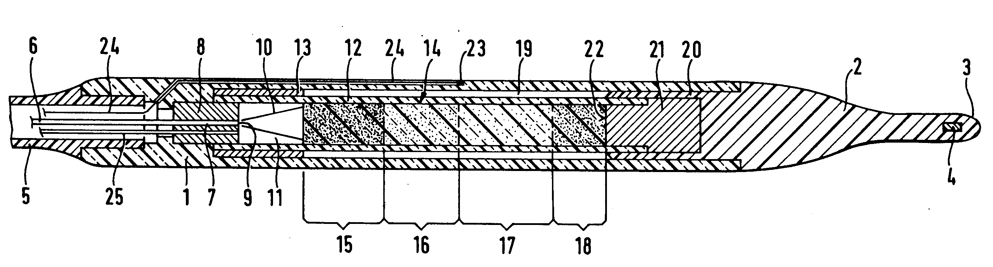

Nachfolgend wird ein Ausführungsbeispiel der Erfindung anhand der Zeichnung näher beschrieben, die in einer einzigen Figur den erfindungsgemässen Lichtdiffusor darstellt, der eine simultane photodynamische Therapie und Hyperthermie im Oesophagus gestattet.

Der in der Zeichnung dargestellte Lichtdiffusor verfügt über ein hülsenförmiges Sondengehäuse 1, das als PMMA-Zylinder mit einem Aussendurchmesser von 15 bis 18 mm und einer Gesamtlänge von etwa 77 bis 117 mm ausgebildet ist. Wie man in der Zeichnung erkennen kann, ist das in der Zeichnung rechts dargestellte vordere Ende mit einem Savary-Gillard Dilatationsstück 2 verbunden, das die in der Zeichnung dargestellte, abgerundete und in eine Spitze 3 auslaufende Form hat, die ein schonendes Einführen des Lichtdiffusors in den Oesophagus eines Patienten gestattet, der von einem Tumor befallen ist und mit Hilfe der photodynamischen Therapie behandelt werden soll.

Um die Lage des Lichtdiffusors in einem Röntgenbild beobachten zu können, ist in der Nähe der Spitze 3 ein Röntgenmarkierer 4 vorgesehen.

An seinem hinteren Ende ist das Sondengehäuse 1 mit einem Nylonschlauch 5 verbunden, der auf seinem Mantel in der Zeichnung nicht erkennbare Längenmarkierungen aufweist, um die Vorführtiefe des Lichtdiffusors überwachen zu können. Durch das Innere des Nylonschlauches 5 verläuft eine optische Faser 6, die an dem in der Zeichnung nicht dargestellten Einspeiseende mit dem Licht eines Lasers beaufschlagt wird. Zur photodynamischen Therapie kann dabei ein Laser mit einer Lichtwellenlänge von 0,630 µm vorgesehen sein. Bei einer Therapie durch eine Hyperthermie wird Laserlicht mit einer Wellenlänge von beispielsweise 1,064 µm in die optische Faser 6 eingespeist. Es ist bekannt, dass Licht dieser grösseren Wellenlänge tiefer in das Gewebe eines Patienten eindringt und zum relativ homogenen Aufwärmen verwendet werden kann.

Das vordere Ende 7 der optischen Faser 6 ist in einem im Sondengehäuse 1 axial unverschiebbar arretierten Haltestück 8 befestigt, durch das es sich koaxial erstreckt. Die Stirnfläche 9 der optischen Faser 6 fluchtet in etwa mit der in der Zeichnung nach rechts weisenden Stirnfläche des Haltestücks 8 und gibt einen aufgeweiteten Strahl 10 ab, der sich durch einen Hohlraum 11 in Richtung des vorderen Endes des Lichtdiffusors erstreckt.

Wie man in der Zeichnung erkennen kann, befindet sich im Innern des Sondengehäuses 1 ein koaxial zum Sondengehäuse 1 verlaufendes Rohr 12 aus PTFE, in dessen in der Zeichnung links liegendes hinteres Ende das Haltestück 8 eingreift. Der hintere Bereich des Rohres 12 ist von einem Aluminiumring 13 umgeben, dessen Innenmantel auftreffendes Licht zurückspiegelt. In Richtung zum vorderen Ende des Rohres erstreckt sich der Aluminiumring 13 in etwa bis zu der Stelle, wo der aufgeweitete Strahl 10 auf eine Silikonfüllung 14 im Rohr 12 auftrifft. An dieser Stelle hat der aufgeweitete Strahl 10 seinen grössten Durchmesser erreicht, der geringfügig kleiner als der Innendurchmesser des Rohres 12 ist. Die Silikonfüllung 14 enthält Quarzkörner oder Quarzpartikel mit einem Durchmesser von 40 µm. Die in die Silikonfüllung 14 eingebetteten Quarzkörner bewirken eine Refraktion infolge einer Brechung des einfallenden Lichtes am Uebergang zwischen dem Quarz und dem Silikon aufgrund der unterschiedlichen Brechungsindizes dieser Materialien. Diese Refraktion ist verhältnismässig wenig wellenlängenabhängig, so dass die mit Quarzkörnern durchsetzte Silikonfüllung 14 in das Rohr 12 axial einfallendes Licht nur wenig wellenlängenabhängig in radialer Richtung streuen kann.

Das konzentrisch im Sondengehäuse 1 angeordnete Rohr 12 weist vorzugsweise eine Silikonfüllung 14 mit in axialer Richtung sich verändernden Quarzkörnerkonzentrationen auf.

In einem ersten Abschnitt 15 mit einer Länge von etwa 12 mm befindet sich eine grosse Konzentration von Quarzkörnern, wobei die Konzentration etwa 0,3 Gewichtsprozent betragen kann.

An den ersten Abschnitt 15 schliesst sich ein zweiter Abschnitt 16 von 11 mm Länge an, der eine geringe Quarzkörnerkonzentration von etwa 0,1 Gewichtsprozent aufweist. Ein nachfolgender dritter Abschnitt 17 einer Länge von 14 mm hat ebenfalls eine verhältnismässig sehr kleine Quarzkörnerkonzentration von etwa 0,06 Gewichtsprozent. In einem vierten Abschnitt 18 mit einer Länge von 8 mm befinden sich in der Silikonfüllung 14 Quarzpartikel von einem Durchmesser mit 40 µm mit einer mittleren Konzentration von etwa 0,25 Gewichtsprozent. Bei einer derartigen Konzentrationsverteilung in axialer Richtung ergibt sich eine sehr hohe homogene Lichtdosierung im Oesophagus des Patienten, wobei sowohl kurzwelliges Licht für eine photodynamische Therapie als auch längerwelliges Licht für eine Hyperthermie gut gestreut wird. Dabei ist es von Vorteil, dass das Silikon eine hohe Transparenz und eine gute Alterungsbeständigkeit aufweist.

Zwischen dem Aussenmantel des Rohres 12 und dem Innenmantel des Sondengehäuses 1 befindet sich ein Ringraum 19, der sich zwischen dem Aluminiumring 13 und einem vorderen Verschlussteil 20 erstreckt.

In das vordere Ende des Rohres 12 ragt ein Aluminiumzylinder 21 hinein, dessen in der Zeichnung nach links weisende Stirnfläche 22 als Spiegel ausgebildet ist und zur Homogenisierung der Lichtverteilung beiträgt.

Im mittleren Bereich des Sondengehäuses 1 befindet sich in dessen Mantel ein sehr kleines Thermoelement 23, das lediglich einen kleinen Schatten wirft, wobei dieser Schatten infolge der Diffusion des Lichtdiffusors und des bestrahlten Gewebes weitgehend ausgeglichen wird. Das Thermoelement 23 ist über eine Leitung 24 mit einer in der Zeichnung nicht dargestellten thermischen Ueberwachungseinrichtung verbunden. Eine ebenfalls in der Zeichnung nicht dargestellte Lichtintensitätssteuer- und Ueberwachungseinrichtung ist mit einer Lichtüberwachungsfaser 25 verbunden, die sich wie die Leitung 24 und die optische Faser 6 durch den Nylonschlauch 5 erstreckt und mit ihrer Stirnfläche in der Nähe der Stirnfläche 9 der optischen Faser 6 endet. Auf diese Weise gestattet es die Lichtüberwachungsfaser 25 die Intensität des Lichtes im Hohlraum 11 und damit die zur Bestrahlung des Patienten verwendete Lichtintensitt zu Ueberwachen und zu steuern.

Das Sondengehäuse 1 kann auf seiner Innenseite entlang der Hälfte seines Umfangs gespiegelt sein, um auf diese Weise zu erreichen, dass der Lichtdiffusor in radialer Richtung nicht über 360 Grad sondern lediglich über 180 Grad Licht aussendet. Die Verspiegelung kann dabei unmittelbar auf der Innenseite des Sondengehäuses 1 aufgebracht sein oder auf ein zusätzliches Teil das in den Ringraum 19 eingebracht ist und rinnenförmig verspiegelt ist.

Statt der oben erwähnten Quarzkörner können auch andere transparente Körner mit einem Brechungsindex, der vom Brechungsindex des Silikons verschieden ist, verwendet werden. Insbesondere können die Körner mit einem Durchmesser von etwa 40 µm aus Glas, Aluminiumoxid, Bariumfluorid, Calziumfluorid und Flintglas bestehen.

Statt des Silikons können auch andere Kunststoffe oder Massen mit entsprechender Transparenz und Beständigkeitseigenschaften verwendet werden.

## Patentansprüche

1. Lichtdiffusor für eine photodynamische Therapie von Tumoren im Oesophagus eines Patienten mit einer optischen Faser (6) zum Einspeisen von Laserlicht in ein das axial einfallende Licht radial umlenkende und in eine Masse (14) eingebettete Partikel enthaltendes Rohr (12), das an dem der optischen Faser (6) gegenüberliegenden vorderen Ende einen Spiegel (22) aufweist und koaxial in einem hülsenförmigen Sondengehäuse (1) angeordnet ist, dadurch gekennzeichnet, dass die das Licht (10) umlenkenden Partikel Körner mit einem gegenüber der Lichtwellenlänge von Laserlicht grossen Durchmesser und einem vom Brechungsindex der Masse verschiedenen Brechungsindex sind.

2. Lichtdiffusor nach Anspruch 1, dadurch gekennzeichnet, dass die Körner Quarzkörner mit einem Durchmesser von etwa 40 µm sind.

3. Lichtdiffusor nach Anspruch 1 oder 2, dadurch gekennzeichnet, dass das Rohr (12) in axialer Richtung aufeinanderfolgende Abschnitte (15 bis 18) mit unterschiedlichen Konzentrationen für die Quarzkörner aufweist.

4. Lichtdiffusor nach Anspruch 3, dadurch gekennzeichnet, dass der vom Laserlicht (10) direkt beaufschlagte erste Abschnitt (15) eine grössere Quarzkörnerkonzentration aufweist als die nachfolgenden Abschnitte (16 bis 18).

5. Lichtdiffusor nach Anspruch 4, dadurch gekennzeichnet, dass sich in axialer Richtung an den ersten Abschnitt (15) hoher Quarzkörnerkonzentration wenigstens ein Abschnitt (16,17) mit einer kleinen Quarzkörnerkonzentration und in axialer Richtung daran anschliessend ein letzter Abschnitt (18) mit mittlerer Quarzkörnerkonzentration anschliesst.

6. Lichtdiffusor nach Anspruch 5, dadurch gekennzeichnet, dass zwei Abschnitte (16,17) jeweils sehr Kleiner und kleiner Quarzkörnerkonzentration im mittleren Bereich zwischen dem ersten Abschnitt (15) und dem letzten Abschnitt (18) vorgesehen sind.

7. Lichtdiffusor nach einem der vorstehenden Ansprüche, dadurch gekennzeichnet, dass im Mantel des Sondengehäuses (1) ein Thermoelement (23) vorgesehen ist.

8. Lichtdiffusor nach einem der vorstehenden Ansprüche, dadurch gekennzeichnet, dass eine Lichtüberwachungsfaser (25) vorgesehen ist, deren Stirnfläche seitlich versetzt neben der optischen Faser (6,9) angeordnet ist und mit einem Teil des von dem hinteren Ende des Rohres (12) zurückkehrenden Lichtes beaufschlagt ist.

9. Lichtdiffusor nach Anspruch 7, dadurch gekennzeichnet, dass das Thermoelement (23) an eine thermische Ueberwachungseinrichtung angeschlossen ist.

10. Lichtdiffusor nach Anspruch 8, dadurch gekennzeichnet, dass die Lichtüberwachungsfaser an eine Lichtintensitätssteuer- und Ueberwachungseinrichtung angeschlossen ist.

11. Lichtdiffusor nach einem der vorstehenden Ansprüche, dadurch gekennzeichnet, dass die Masse Silikon (14) ist.

## Claims

1. A light diffuser for the photodynamic therapy of tumours in the oesophagus of a patient, which diffuser has an optical fibre (6) for feeding laser light into a tube (12) which deflects the axially incident light radially and contains particles embedded in a composition (14) and which has a mirror (22) at the front end, arranged opposite the optical fibre (6), and is arranged coaxially in a sleeve-shaped probe housing (1), wherein the particles deflecting the light (10) are grains that have a large diameter relative to the light wavelength of laser light and have a refractive index that differs from the refractive index of the composition.

2. A light diffuser according to claim 1, wherein the grains are quartz grains having a diameter of approximately 40 µm.

3. A light diffuser according to claim 1 or claim 2, wherein the tube (12) has portions (15 to 18) that follow one another in the axial direction and have different concentrations of quartz grains.

4. A light diffuser according to claim 3, wherein the first portion (15), which is struck directly by the laser light (10), has a greater concentration of quartz grains than have the following portions (16 to 18).

5. A light diffuser according to claim 4, wherein the first portion (15), which has a high concentration of quartz grains, is adjoined in the axial direction by at least one portion (16, 17) having a low concentration of quartz grains, and that portion is adjoined in the axial direction by a final portion (18) having an intermediate concentration of quartz grains.

6. A light diffuser according to claim 5, wherein two portions (16, 17) having a very low and a low concentration of quartz grains, respectively, are provided in the middle region between the first portion (15) and the final portion (18).

7. A light diffuser according to any one of the preceding claims, wherein a thermoelement (23) is provided in the surface of the probe housing (1).

8. A light diffuser according to any one of the preceding claims, wherein a light-monitoring fibre (25) is provided, the end face of which is arranged in a laterally offset manner near the optical fibre (6, 9) and is struck by some of the light returning from the rear end of the tube (12).

9. A light diffuser according to claim 7, wherein the thermoelement (23) is connected to a thermal monitoring device.

10. A light diffuser according to claim 8, wherein the light-monitoring fibre is connected to a light-intensity control and monitoring device.

11. A light diffuser according to any one of the preceding claims, wherein the composition is silicone (14).

## Revendications

1. Diffuseur de lumière pour une thérapie photo-dynamique de tumeurs dans l'oesophage d'un patient, ce diffuseur comportant une fibre optique (6) pour l'introduction de lumière laser dans un tube (12) déviant radialement la lumière d'incidence axiale et contenant des particules incorporées dans une composition ou masse (14), lequel tube présente un miroir (22) à l'extrémité avant située en face de la fibre optique (6) et est disposé dans un corps (1) de sonde en forme de fourreau, caractérisé en ce que les grains de particules déviant la lumière (10) ont un plus grand diamètre que la longueur d'onde de la lumière et ont un indice de réfraction différent de l'indice de réfraction de la composition ou masse.

2. Diffuseur de lumière selon la revendication 1, caractérisé en ce que les grains sont des grains de quartz d'un diamètre d'environ 40 µm.

3. Diffuseur de lumière selon la revendication 3, caractérisé en ce que le tube (12) présente des parties (15 à 18) qui se suivent dans la direction axiale, et dont les concentrations en grains de quartz sont différentes.

4. Diffuseur de lumière selon la revendication 3, caractérisé en ce que la première partie (15), frappée directement par la lumière laser (10), présente une plus grande concentration en grains de quartz que les parties (16 à 18) suivantes.

5. Diffuseur de lumière selon la revendication 4, caractérisé en ce qu'à la première partie (15) à forte concentration en grains de quartz fait suite dans la direction axiale au moins une partie (16, 17) comportant une faible concentration en grains de quartz, et en ce que, faisant suite à celle-ci dans la direction axiale, se trouve une dernière partie (18) à concentration moyenne en grains de quartz.

6. Diffuseur de lumière selon la revendication 5, caractérisé en ce que deux parties (16, 17), l'une de concentration très faible en grains de quartz, l'autre de. faible concentration en grains de quartz, sont prévues dans la zone médiane entre la première partie (15) et la dernière partie (18).

7. Diffuseur de lumière selon l'une des revendications précédentes, caractérisé en ce qu'un élément thermique (23) est prévu dans l'enveloppe du corps (1) de sonde.

8. Diffuseur de lumière selon l'une des revendications précédentes, caractérisé en ce qu'est prévue une fibre (25) de surveillance de la lumière, fibre dont la surface frontale est disposée avec un décalage latéral à côté de la fibre optique (6, 9) et qui est soumise à une partie de la lumière revenant de l'extrémité arrière du tube (12).

9. Diffuseur de lumière selon la revendication 7, caractérisé en ce que l'élément thermique (23) est raccordé à un dispositif de surveillance thermique.

10. Diffuseur de lumière selon la revendication 8, caractérisé en ce que la fibre de surveillance de la lumière est raccordée à un dispositif de commande et de surveillance de l'intensité de la lumière.

11. Diffuseur de lumière selon l'une des revendications précédentes, caractérisé en ce que la composition ou masse est constituée par de la silicone (14).
